# EUROPEAN PATENT SPECIFICATION

(11) **EP 1 195 160 B1**
(45) Date of publication and mention of the grant of the patent: **16.09.2009**
(21) Application number: 00308774.9
(22) Date of filing: 05.10.2000
(51) Int. Cl.: A61K 31/495, A61K 9/20, A61K 9/22, A61P 9/10

(54) **Sustained release trimetazidine pharmaceutical compositions and a method of their preparation**
Trimetazidine enthaltendes Arzneimittel mit verzögerter Wirkstoffabgabe und Verfahren zur Herstellung
Composition pharmaceutique à effet de retard contenant de la trimétazidine et procédé pour sa préparation

(43) Date of publication of application: 10.04.2002
(73) Proprietor: USV Ltd., Govandi, Mumbai 4000 88 (IN)
(72) Inventor: Gidwani, Suresh Kumar, Govandi, Mumbai 4000 88 (IN); Singnurkar, Purushottam S., Govandi, Mumbai 4000 88 (IN); Tewari, Prashant Kumar, Govandi, Mumbai 4000 88 (IN)
(74) Representative: Beresford, Keith Denis Lewis

(56) References cited:
- EP-A- 0 189 861
- EP-A- 0 673 649
- EP-A- 1 108 424
- GB-A- 929 252
- US-A- 4 132 753
- US-A- 4 428 951
- US-A- 5 807 583
- US-A- 5 849 240
- DATABASE WPI Section Ch, Week 198644 Derwent Publications Ltd., London, GB; Class A96, AN 1986-289031 XP002161745 & JP 61 212517 A (NIPPON CHEMIPHAR CO), 20 September 1986 (1986-09-20)

## Description

The present invention relates to sustained release matrix pharmaceutical compositions containing 60mg of trimetazidine dihydrochloride and hydrophobic polymers and/or other hydrophobic materials as a retardant, which release trimetazidine in a sustained and reproducible manner over a prolonged period of time to achieve the sustained effect of trimetazidine over a 24 hour period after oral administration.

Trimetazidine dihydrochloride [1-(2,3,4-trimethoxybenzyl)-piperazine dihydrochloride] composed of formula 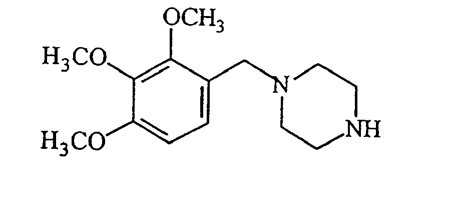 is freely soluble in water, about 80%. It has two pKa values 4.32 and 8.95. It regulates ionic and extra cellular exchanges, correcting the abnormal flow of ions across the cell membrane caused by ischemia and preventing cellular edema caused by anoxia. Thus it ensures the functioning of the ion pumps and the sodium-potassium transmembrane flux and maintains the cellular homeostasis.

Trimetazidine dihydrochloride is used therapeutically, as a coronary vasodilator for the prophylactic treatment of anginal chest pain attack and during such attacks, during chorioretinal attacks as well as for the treatment of giddiness of vascular origin (Vertigo of Maniere, acouphenous).

Trimetazidine dihydrochloride is administered orally in doses of 40 to 60mg daily in divided doses as an immediate release preparation. It is quickly absorbed and eliminated by the organism with plasma half life of around 6.0 ± 1.4 hours and T max of around 1.8 ± 0.7 hours. Since it has a shorter plasma half life, in practice 20mg preparation is given twice or thrice a day in order to ensure relatively constant plasma levels but, due to the fact that it is absorbed quickly, these immediate release forms lead to maximum plasma levels immediately after administration and to a very low plasma level at the time of the next dose, resulting in great differences in peak and trough plasma levels at steady state. Trimetazidine dihydrochloride is regarded as a safe drug in the long treatment of chronic ischemic disorders. This compels the necessity of fabricating the immediate release dosage form into a sustained release once-a-day preparation for achieving regular and constant plasma levels, which is also favourable for compliance of the patient to his treatment.

U.S. Patent 4,814,176 and U.S. Patent 4,755,544 by Makino, Yuji; Matugi, Hideo; Suzuki, Yoshiki; describes a sustained release preparation comprising a) chitin, chitosan or a mixture thereof or a) non-anionic cellulose ether, and b) anionic polymer compounds (Ganterz®) such as those having a carboxyl group, a sulfonic group or a group capable of providing the same. It mentions trimetazidine as one of the examples in the description which can be included in such systems but does not provide any detailed study on trimetazidine as a sustained release preparation.

European Patent Application No. 1108424, which forms part of the state of the art by virtue of Article 54(3) EPC, discloses matrix compositions of trimetazidine dihydrochloride for prolonged release of trimetazidine prepared using standard procedures such as wet granulation, dry granulation or direct compression. Oral dosage forms are disclosed in which the prolonged release of trimetazidine is attributed to the use of cellulose derivatives in the matrix, preferably hydroxypropylmethyl cellulose. JP 61-212517 A discloses a long-acting tablet containing trimetazidine hydrochloride, an enteric polymer base and a hardened oil.

European Patent Application No. 0 673 649 by Huet de Barochez describes pharmaceutical compositions for the prolonged release of trimetazidine or of one of its pharmaceutically acceptable salts characterized in that prolonged release of trimetazidine is controlled by the use of a mixture of water insoluble polymer and a plasticizer coated on a reservoir system containing 80mg of trimetazidine dihydrochloride. However the *in-vivo* bioavailability study conducted on 12 volunteers shows steady state plasma concentration (Css) at around 110ng/ml with 80mg dose.

As reported in the literature, the maximum dose which usually should be formulated in a sustained release dosage form should not exceed the total dose administered by conventional forms during the maintenance period. Optimization of sustained release dosage form design requires minimization of the total dose delivered and maximization of the duration of the drug release. However, European Patent Application No. 0 673 649 describes 80mg dose for prolonged release which is quite high compared to the total conventional dose, 40 to 60mg, in divided doses.

In the present invention the same required steady state plasma concentration of trimetazidine can be achieved with just 60mg dose fabricated in a sustained release once-a-day matrix composition.

Catherine Harpey, Pascale Clauser, Claude Labrid, Jean-Louis Freyria (Cardiovascular Drug Reviews, 1989), determined the pharmacokinetic parameters after single and repeated doses of 20mg trimetazidine dihydrochloride. Their study shows steady state plasma concentration of 84.8 ± 14.8ng/ml with repeated b.i.d. and maximum plasma concentration Cₘₐₓ of 53.6 ± 9.0ng/ml with single dose administration.

Since a sustained release dosage form should give approximately the same steady state plasma concentration as that achieved with an immediate release dosage form, it has been decided to achieve Css around 84ng/ml with the sustained release compositions of the present invention.

The present invention is based on the scientific calculation of the dose of trimetazidine dihydrochloride desired, based on the data available from *in-vivo* studies which are well documented in the scientific literature. The model used here is based on the mathematical equations provided by Dobrinska and Welling (1975) which give fairly accurate calculations about loading dose and maintenance dose for achieving sustained release effect.

The dose of trimetazidine dihydrochloride is calculated by considering the following pharmacokinetic values from the literature.

| | |
|---|---|
| Plasma concentration | Css = 84ng/ml |
| Elimination half life | t 1/2 = 6.0 hours. |
| Volume of distribution | Vd = 294 litres. |
| T max = 1.8 ± 0.7 hours. | |

Using the Dobrinska and Welling model, the calculated loading dose is 15.876mg, the maintenance dose is 44.008mg and the total dose is 59.88mg of trimetazidine dihydrochloride for achieving a sustained release effect for 24 hours.

The object of the present invention is to prepare a composition containing 60mg of trimetazidine dihydrochloride by suitable technology showing demonstrable release rate and facilitated *in-vivo* absorption for the desired period. The emphasis is to develop a simple monolithic system composed of hydrophobic polymers and/or other hydrophobic materials and other excipients with improved kinetics of extended release dosage forms, and the simplest method of producing it.

The monolithic (e.g. tablet form) sustained release system produced by the method the invention is a homogeneous system composed of active drug preferably in an amount within the range of 8 to 50% by weight, more preferably 10 to 30% by weight, and one or more hydrophobic polymers and/or one or more other types of hydrophobic material preferably in an amount within the range of about 20 to 80% by weight, more preferably 30 to 60% by weight, based on the weight of the pharmaceutical composition.

The hydrocolloid forming material which may be employed for the monolithic sustained release system includes, but is not limited to: hydroxypropyl methyl cellulose (e.g. Methocel®), hydroxy propyl cellulose (e.g. Klucel®), polyethyleneoxide (average molecular weight 600,000 to 5,000,000); sodium carboxymethyl cellulose, calcium carboxymethyl cellulose, calcium ammonium alginate, sodium alginate, potassium alginate, calcium alginate, propylene glycol alginate, alginic acid, polyvinyl alcohol, povidone, carbomer, potassium pectate, guar gum, xanthane gum. Mixtures of the hydrocolloid forming material may be employed in a weight ratio to another hydrocolloid forming material within the range of about 1: 0.1 to 0.1 : 1, preferably about 1 : 0.5 to 0.5 : 1

Hydrophobic polymers which may be employed for the monolithic sustained release system include, but are not limited to: stearic acid, glycerylmonostearate, glyceryl behenate, glyceryl monooleate, glyceryl palmitostearate, microcrystalline wax, stearyl alcohol, cetyl alcohol, cetostearyl alcohol, hydrogenated castor oil, tristearin, waxes, polyvinyl alcohol, polyethylene powder, polyvinyl chloride, shellac, rosin, and the like. Mixtures of the hydrophobic polymer may be employed in a weight ratio to another hydrophobic material within the range of about 1: 0.1 to 0.1 : 1, preferably about 1 : 0.5 to 0.5 : 1.

Mixtures of hydrocolloid forming material with hydrophobic polymer and/or other hydrophobic material may also be used in the method of producing pharmaceutical compositions according to the present invention and may be employed in the weight ratio ranging from 1 : 0.1 to 0.1 : 1, preferably 1 : 0.5 to 0.5 : 1

The pharmaceutical compositions produced using the method according to the present invention may be used to form compressed tablets of any shape, preferably of round shape, and can be additionally provided with a film coat of commonly used hydrophilic coating polymers. The film envelope used can be a taste neutralizing film forming agent, to which dyes may optionally be added for elegance. The proportion by weight of the film envelope relative to the final tablet is in the usual range of 0.5 to 4.0% by weight, preferably 1.0 to 1.5% by weight. Film formers such as hydroxypropyl methylcellulose, hydroxypropyl cellulose, starch, cellulose derivatives and the like may be used.

The monolithic composition can also be used to produce compressed slugs and filled into capsules.

Auxiliary substances employed in the method of the present invention for producing the monolithic sustained release system include diluent, including but not limited to: dibasic calcium phosphate, tribasic calcium phosphate, calcium carbonate, lactose, etc.; binder including but not limited to: polyvinyl pyrrolidone, gelatin, gum acacia, Klucel EF (hydroxypropyl cellulose), carboxymethyl cellulose sodium, etc., glidants including but not limited to: colloidal silicone dioxide, talc, starch, and the like; lubricants including but not limited to: magnesium stearate, zinc stearate, and the like.

The pharmaceutical dosage form, such as tablet, produced by the method according to the present invention, apart from active drug and hydrocolloid forming materials and/or hydrophobic polymers and or hydrophobic materials, may contain 10 to 70% by weight, preferably 20 to 60% by weight, of diluent; 1.0 to 15% by weight of a binder, preferably 2.0 to 10% by weight; and up to 3.0% by weight of glidant, preferably 0.5 to 1.5% by weight; and up to 2.0% by weight of lubricants, preferably 0.5 to 1.0% by weight; each in relation to the weight of pharmaceutical composition.

According to the method of the present invention the pharmaceutical composition, such as tablets, is produced by dry mixing of active substance and optionally further auxiliary substance and granulating this mixture with hydrophobic polymers and/or other hydrophobic material (s) by a hot melt granulation technique using a jacketed rapid mixer granulator at a temperature of 40 to 120°C, preferably 60 to 80°C. This is followed by gradual cooling of the granulate mass to room temperature with continuous mixing. The resulting mass is further granulated with aqueous or organic solution of the binder, followed by drying and converting it into granules of 30µm to 2.0mm, preferably 100µm to 1.0mm, by milling and sizing.

Subsequently appropriate other pharmaceutical auxiliary substances are admixed with the sized granules. The composition produced in this manner is subsequently processed in the usual manner to produce pharmaceutical dosage forms, such as by compression into tablets or by the filling of pressed slugs into capsules. The tablets can be coated with a film using standard coating processes and methods such as a conventional coating pan or fluid coating process.

The sustained release tablets obtained using the method according to the present invention release trimetazidine dihydrochloride in a controlled manner which provides an effect over a time period up to 24 hours, preferably over 18 hours as per the calculations.

Useful trimetazidine sustained release formulations produced according to the invention show the following *in-vitro* drug release characteristics when tested in gastric fluid pH 1.2 for the first hour and then in phosphate buffer pH 6.8 USP.

| **Time** | **% Release** |
|---|---|
| 1 | 30 - 34 % |
| 2 | 42 - 48 % |
| 3 | 52 - 58 % |
| 4 | 62 - 68 % |
| 5 | 72 - 78 % |
| 6 | 78 - 82 % |
| 7 | 82 - 86 % |
| 8 | 85 - 88 % |
| 9 | 87 - 92 % |
| 10 | 91 - 94 % |
| 11 | 93 - 96 % |
| 12 | 95 - 99 % |

### Background Example 1 :

600g of trimetazidine dihydrochloride, 1840g of Methocel E4M CR (HPMC), 2098g of anhydrous dibasic calcium phosphate were mixed and granulated in a solution containing 100g polyvinyl pyrrolidone dissolved in 2250ml of isopropyl alcohol. The resulting mass was dried at 45°C and the agglomerates were sized through a 2.4mm screen. These sized granules (4638g) were blended with 14g of colloidal silicone dioxide and 48g of magnesium stearate and compressed into round tablets each containing 60mg of trimetazidine dihydrochloride.

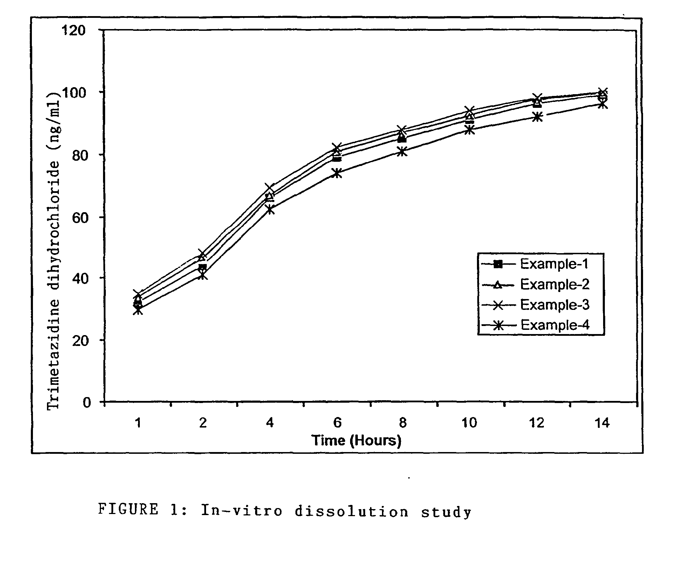

The *in-vitro* release of trimetazidine dihydrochloride from these tablets is shown in Figure 1.

### In-vivo bioavailability study:

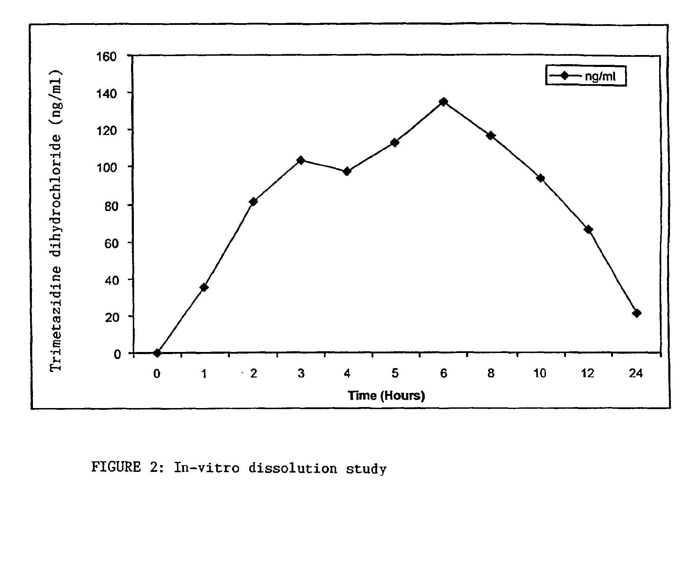

*In-vivo* bioavailability studies were carried out on 6 healthy human volunteers after oral administration of Trimetazidine SR tablets of Example 1 containing 60mg of trimetazidine dihydrochloride. The plasma trimetazidine concentration versus time over 24 hours is shown in Figure 2.

### Conclusion:

Single dose *in-vivo* bioavailability study shows well sustained plasma levels of trimetazidine over 24 hours with around 20ng/ml plasma concentration of trimetazidine after 24 hours with maximum plasma concentration of 135ng/ml.

### Background Example 2 :

600g of trimetazidine dihydrochloride, 1840g of Methocel E4M CR (HPMC), 2098g of anhydrous dibasic calcium phosphate were mixed and granulated in a solution containing 100g polyvinyl pyrrolidone dissolved in 2250 ml of isopropyl alcohol. The resulting mass was dried at 45°C and the agglomerates were sized through a 2.4mm screen. These sized granules (4638g) were blended with 14g of colloidal silicone dioxide and 48g of magnesium stearate and compressed into compact slugs and filled into capsules, each containing 60mg of trimetazidine dihydrochloride.

The *in-vitro* release of trimetazidine dihydrochloride from these tablets is shown in Figure 1.

### Background Example 3 :

600g of trimetazidine dihydrochloride, 2000g of Klucel GF (HPC), 2098g of anhydrous dibasic calcium phosphate were mixed and granulated in a solution containing 100g polyvinyl pyrrolidone dissolved in 2250 ml of isopropyl alcohol. The resulting mass was dried at 45°C and the agglomerates were sized through a 2.4mm screen. These sized granules (4798g) were blended with 14g of colloidal silicone dioxide and 48g of magnesium stearate and compressed into round tablets, each containing 60mg of trimetazidine dihydrochloride.

The *in-vitro* release of trimetazidine dihydrochloride from these tablets is shown in Figure 1.

### Background Example 4 :

600g of trimetazidine dihydrochloride, 1500g of Methocel E4M CR (HPMC), 380g of carboxymethyl cellulose sodium and 2050g of anhydrous dibasic calcium phosphate were mixed and granulated in a solution containing 100g polyvinyl pyrrolidone dissolved in 2250ml of isopropyl alcohol. The resulting mass was dried at 45°C and the agglomerates were sized through a 2.4mm screen. These sized granules (4630g) were blended with 14g of colloidal silicone dioxide and 48g of magnesium stearate and compressed into round tablets, each containing 60mg of trimetazidine dihydrochloride.

The *in-vitro* release of trimetazidine dihydrochloride from these tablets is shown in Figure 1.

### Example 5 :

360g of stearic acid was melted at 70°C. 600g of trimetazidine dihydrochloride and 1000g of lactose monohydrate were mixed and heated to 70°C in a jacketed rapid mixer granulator and granulated with the above melted stearic acid at 70°C. After granulation, the granulated mass was mixed continuously with gradual cooling to room temperature.

100g of shellac and 50g of polyvinyl pyrrolidone were dissolved in 250g of isopropyl alcohol. This solution was gradually added to the above trimetazidine stearic acid and lactose granulate and mixed till a dough mass formed. The resulting dough mass was dried at 45°C for 2 hours and then sized through 2.4mm screen to break the agglomerates. These sized granules (2110g) were blended with 10g of colloidal silicone dioxide and 20g of magnesium stearate and compressed into round tablets each containing 60mg of trimetazidine dihydrochloride.

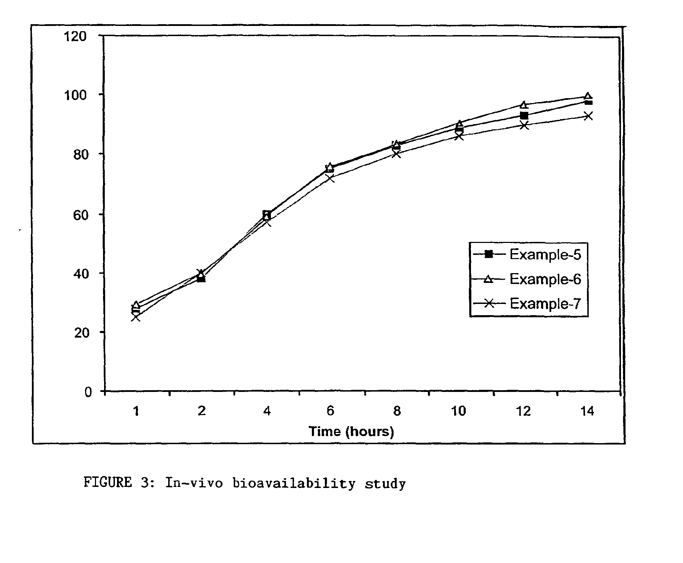

The *in-vitro* release of trimetazidine dihydrochloride from these tablets is shown in Figure 3.

### Example 6 :

400g of glyceryl monostearate was melted at 60°C. 600g of trimetazidine dihydrochloride and 1000g of lactose monohydrate were mixed and heated to 60°C in a jacketed rapid mixer granulator and granulated with the above melted stearic acid at 60°C. After granulation, the granulated mass was mixed continuously with gradual cooling to room temperature.

100g of shellac and 50g of polyvinyl pyrrolidone were dissolved in 250g of isopropyl alcohol. This solution was gradually added to the above trimetazidine stearic acid and lactose granulate and mixed till a dough mass formed. The resulting dough mass was dried at 45°C for 2 hours and then sized through a 2.4mm screen to break the agglomerates. These sized granules (2150g) were blended with 10g of colloidal silicone dioxide and 20g of magnesium stearate and compressed into round tablets - each containing 60g of trimetazidine dihydrochloride.

The *in-vitro* release of trimetazidine dihydrochloride from these tablets is shown in Figure 3.

### Example 7 :

120g of glyceryl monostearate was melted at 60°C. 600g of trimetazidine dihydrochloride was heated to 60°C in a jacketed rapid mixer granulator and granulated with the above melted glyceryl monostearate at 60°C. After granulation, the granulated mass was mixed continuously with gradual cooling to room temperature.

The resulting granule mass was then sized through a 1.5mm screen to break the agglomerates. These sized granules (720g) were blended with 1650g of methocel E4M-CR (HPMC), 1000g of lactose monohydrate, 10g of colloidal silicone dioxide and 20g of magnesium stearate and compressed into round tablets each containing 60mg of trimetazidine dihydrochloride.

The *in-vitro* release of trimetazidine dihydrochloride from these tablets is shown in Figure 3.

### References :

1. Huet de Barochez, Bruno; [1995] ; European Patent 673 649 Al.
2. Makino, Yuji; Matugi, Hideo; Suzuki, Yoshiki; [1988], U.S. Patent 4,755,544
3. Makino, Yuji; Matugi, Hideo; Suzuki, Yoshiki; [1989] , U.S. Patent 4,814,176.
4. Yoshida Yoshiyuki; Kondo Seiji; Yamasaka Hiranojiyou; Okazawa Heiichi; [1986]; Japanese Patent JP 61212517A.
5. Huet de Barochez, Bruno; Genty Patric; Alain Cuine; [1995]; Japanese Patent JP 7258086A.
6. Catherine Harpey; Pascale Clauser; Claude Labrid; Jean-Louis Freyria; Cardiovascular Drug Reviews; 1989; 6 (4); 292 312.
7. Leon Lachman; Herbet Liberman; Joseph Kanig, The Theory and Practice of Industrial Pharmacy; Lea & Febiger Philadelphia U.S.A.; Third edition; 1987; 430 442.
8. Dobrinska, Welling, J. Pharm. Sciences 1975.

## Claims

1. A method of preparing a sustained release pharmaceutical composition for oral administration of trimetazidine dihydrochloride, comprising:
granulating trimetazidine dihydrochloride with at least one of a hydrophobic polymer and/or other hydrophobic material to form a granulate mass;
**characterised in that** granulation of the mixture is carried out by hot melt granulation at a temperature of 40°C to 120°C.

2. A method as claimed in claim 1, wherein the temperature for hot melt granulation is 60°C to 80°C.

3. A method as claimed in claim 1 or claim 2 further comprising cooling the hot melt granulate mass to room temperature with continuous stirring and further granulating the granulate mass with an aqueous or organic solution of binder, drying it and converting it to granules by milling and sizing.

4. A method as claimed in any preceding claim wherein trimetazidine dihydrochloride constitutes 8 to 50% by weight of the composition.

5. A method as claimed in claim 4 wherein trimétazidine dihydrochloride constitutes 10 to 30% by weight of the composition.

6. A method as claimed in any preceding claim wherein the sustained release dose of trimetazidine is at least 60 mg.

7. A method as claimed in any preceding claim wherein the hydrophobic material/s used is/are selected from the group consisting of fatty acids, fatty alcohols, fatty acid esters, and natural resins.

8. A method as claimed in claim 7 wherein the hydrophobic materials comprise stearic acid, glyceryl monostearate, glyceryl behenate, glyceryl palmitostearate, glyceryl monooleate, microcrystalline wax, stearyl alcohol, cetyl alcohol, cetostearyl alcohol, tristearin, shellac, rosin, polyvinyl chloride powder or polyethylene powder.

9. A method as claimed in claim 3 comprising admixing auxiliary substance with the sized granules.

10. A method as claimed in any preceding claim comprising adding auxiliary substance in a dry mixing step prior to granulation.

11. A method as claimed in claim 9 or claim 10 wherein the auxiliary substance is selected from the group consisting of diluent, binder, glidant, lubricant.

12. A method as claimed in claim 11 wherein the diluent is selected from calcium carbonate or lactose.

13. A method as claimed in claim 12 wherein the diluent comprises 10 to 70% by weight of the composition.

14. A method as claimed in claim 12 wherein the diluent comprises 20 to 60% by weight of the composition.

15. A method as claimed in claim 3 or claim 11 wherein the binder is selected from the group consisting of polyvinyl pyrrolidone, gelatin, gum acacia, hydroxypropyl cellulose or sodium carboxymethyl cellulose.

16. A method as claimed in claim 15 wherein the binder comprises 2 to 10% by weight of the composition.

17. A method as claimed in claim 11 wherein the glidant is selected from the group consisting of colloidal silicone dioxide; talc or starch.

18. A method as claimed in claim 17 wherein the glidant comprises 0.5 to 1.5% by weight of the composition.

19. A method as claimed in claim 11 wherein the lubricant is selected from magnesium stearate or zinc stearate.

20. A method as claimed in claim 19 wherein the lubricant comprises 0.5 to 1.0% by weight of the composition.

21. A method of preparing a sustained release pharmaceutical composition as claimed in any preceding claim wherein the composition is in the form of tablets.

22. A method as claimed in claim 21 further comprising coating the tablets.

23. A method as claimed in claim 22 wherein the coating is a film coating.

24. A method as claimed in claim 23 wherein the coating is a taste neutralizing film.

25. A method as claimed in claim 24 wherein the coating contains dye.

26. A method as claimed in any one of claims 23 to 25 wherein the film coating comprises 0.5 to 4.0% by weight of the tablet.

27. A method as claimed in any one of claims 23 to 26 ,wherein the film coating is formed using hydroxypropyl cellulose, starch or cellulose derivatives.

28. A pharmaceutical composition for oral administration obtained by the method of any preceding claim wherein the trimetazidine is released according to the following dissolution profile:
| Time/Hours | % Release |
|---|---|
| 1 | 30-34% |
| 2 | 42-48% |
| 3 | 52-58% |
| 4 | 62-68% |
| 5 | 72-78% |
| 6 | 78-82% |
| 7 | 82-86% |
| 8 | 85-88% |
| 9 | 87-92% |
| 10 | 91-94% |
| 11 | 93-96% |
| 12 | 95-99% |
when tested in vitro in gastric simulated fluid pH 1.2 for the first hour and then in phosphate buffer pH 6.8 USP.

## Patentansprüche

1. Verfahren zur Herstellung einer pharmazeutischen Zusammensetzung mit verzögerter Wirkstofffreigabe zur oralen Verabreichung von Trimetazidin-Dihydrochlorid, wobei
Trimetazidin-Dihydrochlorid mit einem hydrophoben Polymer und/oder einem anderen hydrophoben Material granuliert wird, um eine Granulatmasse zu bilden,
**dadurch gekennzeichnet, dass** die Granulation der Mischung durch Heißschmelzgranulation bei einer Temperatur von 40°C bis 120°C ausgeführt wird.

2. Verfahren nach Anspruch 1, wobei die Temperatur für die Heißschmelzgranulation zwischen 60°C und 80°C liegt.

3. Verfahren nach Anspruch 1 oder 2, wobei ferner die Heißschmelzgranulatmasse unter fortwährendem Rühren auf Raumtemperatur gekühlt wird und die Granulatmasse ferner mit einer wässrigen oder organischen Lösung eines Bindemittels granuliert, getrocknet und durch Mahlen und Sortieren in Körnchen umgewandelt wird.

4. Verfahren nach einem der vorstehenden Ansprüche, wobei Trimetazidin-Dihydrochlorid 8 bis 50 Gew.-% der Zusammensetzung darstellt.

5. Verfahren nach Anspruch 4, wobei Trimetazidin-Dihydrochlorid 10 bis 30 Gew.-% der Zusammensetzung bildet.

6. Verfahren nach einem der vorstehenden Ansprüche, wobei die verzögert freigegebene Wirkstoffmenge von Trimetazidin wenigstens 60 mg beträgt.

7. Verfahren nach einem der vorstehenden Ansprüche, wobei das verwendete hydrophobe Material bzw. die verwendeten hydrophoben Materialien aus der Gruppe bestehend aus Fettsäuren, Fettalkoholen, Fettsäureestern und Naturharzen ausgewählt ist bzw. sind.

8. Verfahren nach Anspruch 7, wobei die hydrophoben Materialien Stearinsäure, Glycerylmonostearat, Glycerylbehenat, Glycerylpalmitostearat, Glycerylmonooleat, mikrokristallines Wachs, Stearylalkohol, Cetylalkohol, Cetostearylalkohol, Tristearin, Schellack, Kunstharz, Polyvinylchloridpulver oder Polyehtylenpulver umfassen.

9. Verfahren nach Anspruch 3, wobei den sortierten Körnchen eine Hilfssubstanz beigemischt wird.

10. Verfahren nach einem der vorstehenden Ansprüche, wobei eine Hilfssubstanz in einem Trockenmischungsschritt vor der Granulation zugegeben wird.

11. Verfahren nach Anspruch 9 oder 10, wobei die Hilfssubstanz ausgewählt wird aus der Gruppe bestehend aus Verdünnungsmittel, Bindemittel, Gleitmittel und Schmiermittel.

12. Verfahren nach Anspruch 11, wobei das Verdünnungsmittel aus Kalziumkarbonat oder Laktose ausgewählt ist.

13. Verfahren nach Anspruch 12, wobei das Verdünnungsmittel 10 bis 70 Gew.-% der Zusammensetzung ausmacht.

14. Verfahren nach Anspruch 12, wobei das Verdünnungsmittel 20 bis 60 Gew.-% der Zusammensetzung ausmacht.

15. Verfahren nach Anspruch 3 oder 11, wobei das Bindemittel aus der Gruppe bestehend aus Polyvinylpyrrolidon, Gelatine, Gummiarabikum, Hydroxypropylzellulose oder Natriumcarboxymethylzellulose ausgewählt ist.

16. Verfahren nach Anspruch 15, wobei das Bindemittel 2 bis 10 Gew.-% der Zusammensetzung ausmacht.

17. Verfahren nach Anspruch 11, wobei das Gleitmittel aus der Gruppe bestehend aus kolloidalem Silikondioxid, Talk oder Stärke ausgewählt ist.

18. Verfahren nach Anspruch 17, wobei das Gleitmittel 0,5 bis 1,5 Gew.-% der Zusammensetzung ausmacht.

19. Verfahren nach Anspruch 11, wobei das Schmiermittel in Magnesiumstearat oder Zinkstearat besteht.

20. Verfahren nach Anspruch 19, wobei das Schmiermittel 0,5 bis 1,0 Gew.-% der Zusammensetzung ausmacht.

21. Verfahren zur Herstellung einer pharmazeutischen Zusammensetzung mit verzögerter Wirkstofffreigabe gemäß einem der vorstehenden Ansprüche, wobei die Zusammensetzung in Form von Tabletten vorliegt.

22. Verfahren nach Anspruch 21, wobei die Tabletten ferner beschichtet werden.

23. Verfahren nach Anspruch 22, wobei die Beschichtung eine Filmbeschichtung ist.

24. Verfahren nach Anspruch 23, wobei die Beschichtung ein geschmackneutralisierender Film ist.

25. Verfahren nach Anspruch 24, wobei die Beschichtung einen Farbstoff enthält.

26. Verfahren nach einem der Ansprüche 23 bis 25, wobei die Filmbeschichtung 0,5 bis 4,0 Gew.-% der Tablette ausmacht.

27. Verfahren nach einem der Ansprüche 23 bis 26, wobei die Filmbeschichtung unter Verwendung von Hydroxypropylzellulose, Stärke oder Zellulosederivaten gebildet wird.

28. Pharmazeutische Zusammensetzung zur oralen Verabreichung erhältlich durch das Verfahren gemäß einem der vorstehenden Ansprüche, wobei das Trimetazidin gemäß dem folgenden Auflösungsprofil freigegeben wird:
| Zeit/Stunden | % Freisetzung |
|---|---|
| 1 | 30-34% |
| 2 | 42-48% |
| 3 | 52-58% |
| 4 | 62-68% |
| 5 | 72-78% |
| 6 | 78-82% |
| 7 | 82-86% |
| 8 | 85-88% |
| 9 | 87-92% |
| 10 | 91-94% |
| 11 | 93-96% |
| 12 | 95-99% |
gemessen *in vitro* in einem Magensimulationsfluid mit pH 1,2 während der ersten Stunde und dann in einem Phosphatpuffer mit pH 6,8 USP.

## Revendications

1. Procédé de préparation d'une composition pharmaceutique à libération prolongée pour l'administration orale de dichlorhydrate de trimétazidine, comprenant :
la granulation de dichlorhydrate de trimétazidine avec l'un au moins d'un polymère hydrophobe et/ou d'une autre matière hydrophobe pour former une masse de granulat ;
**caractérisé en ce que** la granulation du mélange est effectuée par thermogranulation en masse fondue à une température de 40°C à 120°C.

2. Procédé selon la revendication 1, dans lequel la température pour la thermogranulation en masse fondue est de 60°C à 80°C.

3. Procédé selon la revendication 1 ou la revendication 2, comprenant de plus le refroidissement de la masse de granulat fondue à la température ambiante sous agitation continue et, de plus, la granulation de la masse de granulat avec une solution aqueuse ou organique de liant, son séchage et sa transformation en granules par broyage et calibrage.

4. Procédé selon l'une quelconque des revendications précédentes, dans lequel le dichlorhydrate de trimétazidine constitue 8 à 50 % en poids de la composition.

5. Procédé selon la revendication 4, dans lequel le dichlorhydrate de trimétazidine représente 10 à 30 % en poids de la composition.

6. Procédé selon l'une quelconque des revendications précédentes, dans lequel la dose à libération prolongée de trimétazidine est d'au moins 60 mg.

7. Procédé selon l'une quelconque des revendications précédentes, dans lequel la ou les matière(s) hydrophobe(s) utilisée(s) est/sont choisie(s) dans le groupe formé par les acides gras, les alcools gras, les esters d'acides gras et les résines naturelles.

8. Procédé selon la revendication 7, dans lequel les matières hydrophobes comprennent l'acide stéarique, le monostéarate de glycéryle, le béhénate de glycéryle, le palmitostéarate de glycéryle, le monooléate de glycéryle, la cire microcristalline, l'alcool stéarylique, l'alcool cétylique, l'alcool cétostéarylique, la tristéarine, la gomme-laque, la colophane, une poudre de chlorure de polyvinyle ou une poudre de polyéthylène.

9. Procédé selon la revendication 3, comprenant l'addition et le mélange d'une substance auxiliaire avec les granules calibrés.

10. Procédé selon l'une quelconque des revendications précédentes, comprenant l'addition d'une substance auxiliaire dans une étape de mélange à sec avant la granulation.

11. Procédé selon la revendication 9 ou la revendication 10, dans lequel la substance auxiliaire est choisie dans le groupe formé par un diluant, un liant, un agent de glissement, un lubrifiant.

12. Procédé selon la revendication 11, dans lequel le diluant est choisi parmi le carbonate de calcium ou le lactose.

13. Procédé selon la revendication 12, dans lequel le diluant constitue 10 à 70 % en poids de la composition.

14. Procédé selon la revendication 12, dans lequel le diluant constitue 20 à 60 % en poids de la composition.

15. Procédé selon la revendication 3 ou la revendication 11, dans lequel le liant est choisi dans le groupe formé par la polyvinylpyrrolidone, la gélatine, la gomme arabique, l'hydroxypropylcellulose ou la carboxy-méthylcellulose sodique.

16. Procédé selon la revendication 15, dans lequel le liant constitue 2 à 10 % en poids de la composition.

17. Procédé selon la revendication 11, dans lequel l'agent de glissement est choisi dans le groupe formé par le dioxyde de silicium colloïdal, le talc ou l'amidon.

18. Procédé selon la revendication 17, dans lequel l'agent de glissement constitue 0, 5 à 1,5 % en poids de la composition.

19. Procédé selon la revendication 11, dans lequel le lubrifiant est choisi parmi le stéarate de magnésium ou le stéarate de zinc.

20. Procédé selon la revendication 19, dans lequel le lubrifiant constitue 0,5 à 1,0 % en poids de la composition.

21. Procédé de préparation d'une composition pharmaceutique à libération prolongée selon l'une quelconque des revendications précédentes, dans lequel la composition est sous la forme de comprimés.

22. Procédé selon la revendication 21, comprenant de plus l'enrobage des comprimés.

23. Procédé selon la revendication 22, dans lequel l'enrobage est un enrobage par film.

24. Procédé selon la revendication 23, dans lequel l'enrobage est un film neutralisant le goût.

25. Procédé selon la revendication 24, dans lequel l'enrobage contient une matière colorante.

26. Procédé selon l'une quelconque des revendications 23 à 25, dans lequel le film d'enrobage constitue 0,5 à 4,0 % en poids du comprimé.

27. Procédé selon l'une quelconque des revendications 23 à 26, dans lequel le film d'enrobage est formé en utilisant de l'hydroxypropylcellulose, de l'amidon ou des dérivés de cellulose.

28. Composition pharmaceutique pour l'administration orale, obtenue par le procédé de l'une quelconque des revendications précédentes, dans laquelle la trimétazidine est libérée selon le profil de dissolution suivant :
| Temps/Heures | % de libération |
|---|---|
| 1 | 30-34 % |
| 2 | 42-48 % |
| 3 | 52-58 % |
| 4 | 62-68 % |
| 5 | 72-78 % |
| 6 | 78-82 % |
| 7 | 82-86 % |
| 8 | 85-88 % |
| 9 | 87-92 % |
| 10 | 91-94 % |
| 11 | 93-96 % |
| 12 | 95-99 % |
lorsqu'elle est testée in vitro dans du fluide gastrique simulé à pH 1,2 pendant la première heure, puis dans du tampon phosphate à pH 6,8 USP.
